(19) Europäisches Patentamt
European Patent Office
Office européen des brevets

(11) Veröffentlichungsnummer: **0 154 948**
**B1**

(12) **EUROPÄISCHE PATENTSCHRIFT**

(45) Veröffentlichungstag der Patentschrift: **30.08.89**

(51) Int. Cl.$^4$: **C 07 F 9/40**, A 61 K 7/075

(21) Anmeldenummer: **85102636.9**

(22) Anmeldetag: **08.03.85**

# (54) Phosphonsäureester in Haarbehandlungsmitteln.

(30) Priorität: **16.03.84 DE 3409634**

(43) Veröffentlichungstag der Anmeldung: **18.09.85 Patentblatt 85/38**

(45) Bekanntmachung des Hinweises auf die Patenterteilung: **30.08.89 Patentblatt 89/35**

(84) Benannte Vertragsstaaten: **AT BE CH DE FR GB IT LI LU NL SE**

(56) Entgegenhaltungen:
**DE-B- 1 295 138**
**FR-A- 2 281 376**
**GB-A- 1 183 471**
**US-A- 2 587 340**
**CHEMICAL ABSTRACTS, Band 99, Nr. 24, December 1983, Seite 326, Nr. 200346y, Columbus, Ohio, US; & JP - A - 58 131 912 (SANYO CHEMICAL INDUSTRIES, LTD.) 06-08-1983**

(73) Patentinhaber: **Henkel Kommanditgesellschaft auf Aktien**
**Postfach 1100 Henkelstrasse 67**
**D-4000 Düsseldorf-Holthausen (DE)**

(72) Erfinder: **Ploog, Uwe, Dr.**
**Haydnweg 6**
**D-5657 Haan (DE)**
Erfinder: **Busch, Peter, Dr.**
**Gottfried-August-Bürger-Strasse 10**
**D-4006 Erkrath-Unterbach (DE)**
Erfinder: **Hensen, Hermann, Dr.**
**Tizianweg 4**
**D-4010 Hilden (DE)**
Erfinder: **Thiele, Klaus**
**Rügenweg 5**
**D-4018 Langenfeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Gegenstand der Erfindung sind neue Phosphonsäureester und kosmetische Haarbehandlungsmittel, welche diese Phosphonsäureester als Zusatz zur Verbesserung der Naßkämmbarkeit des Haars enthalten.

Das Haupthaar weist nach dem Waschen mit Shampoos auf Basis von synthetischen oberflächenaktiven Stoffen oft einen kosmetisch unbefriedigenden Zustand auf : Es fühlt sich stumpf an und ist im nassen Zustand nur schwer zu kämmen. Nach dem Trocknen neigt das gewaschene Haar zur statischen Aufladung, wodruch das Kämmen erschwert und der Sitz des gekämmten Haares gestört wird.

Es ist bekannt, nach dem Waschen oder Shamponieren des Haares konditionierende Präparate auf das Haar einwirken zu lassen. Dabei handelt es sich meist um gelförmige, flüssige oder emulsionsförmige Lotionen mit einem Gehalt an kationaktiven grenzflächenaktiven Stoffen. Es ist auch bekannt, üblichen Shampoos bestimmte Substanzen beizufügen, um gleichzeitig mit der Haarwäsche einen gewissen konditionierenden Effekt zu erzielen. Solche Substanzen sind z.B. wasserlösliche Proteine, Proteinabbauprodukte oder polykationische Polymere, z.B. kationische Cellulosederivate. Nachteilig bei den kationaktiven oberflächenaktiven Stoffen ist deren mangelhafte Verträglichkeit mit anionischen Tensiden und deren oft nicht befriedigende Schleimhautverträglichkeit.

Die polykationischen Polymeren wirken der statischen Aufladung des trockenen Haares nicht entgegen, in vielen Fällen wird die Aufladbarkeit des Haares sogar erhöht. Andererseits führt die starke Adsorption dieser kationischen Polymeren an die Keratinfaser, insbesondere bei mehrmaliger Anwendung, zu einer Akkumulation der Polymeren auf dem Haar, welches dadurch « belastet » wird und Elastizität, Sitz und Fülle verliert. Es bestand daher ein Bedürfnis an Haarpflegemitteln, welche die Naßkämmbarkeit des Haares verbesserende Zusätze enthalten und die geschilderten Nachteile nicht oder in geringerem Umfang aufweisen.

Aus US-A-2.587.340 sind Phosphonsäurepolyoxyalkylester als effektive Netzmittel, Emulgatoren, Penetriermittel, Dispergatoren oder Detergentien und ihre Verwendung als Ölzusätze, Textilbehandlungsmittel und Plastifikatoren bekannt.

Aus DE-B-1295138 sind Haarwaschmittelzusammensetzungen bekannt, die Salze von organischen Mono- und Diphosphonsäuren enthalten.

Es wurde gefunden, daß die Naßkämmbarkeit des Haares durch Haarbehandlungsmittel deutlich verbessert wird, die als Zusatz bestimmte Phosphonsäureester enthalten. Diese Zusätze weisen die Nachteile der bekannten, die Naßkämmbarkeit verbessernden Zusätze nicht auf.

Gegenstand der Erfindung ist die Verwendung von Phosphonsäureestern, dieerhältlich sind durch Umsetzung von Organophosphonsäuren der allgemeinen Formel

$$R—PO_3H_2$$

in der R eine lineare oder verzweigte Alkylgruppe mit 2 bis 22 C-Atomen oder eine Gruppe der allgemeinem Formeln I, II oder III

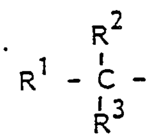 , 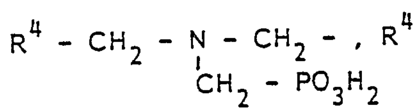 , 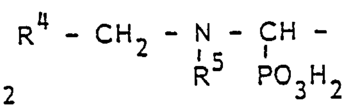

I II III

ist,

in welchen $R^1$ Wasserstoff oder eine Alkylgruppe mit 1 bis 21 C-Atomen, $R^2$ Wasserstoff oder eine $PO_3H_2$-Gruppe, $R^3$ Wasserstoff, eine OH-Gruppe oder, wenn $R^2$ eine $PO_3H_2$-Gruppe ist, eine $NH_2$-Gruppe, $R^4$ eine Gruppe $R^1$ oder eine $PO_3H_2$-Gruppe und $R^5$ Wasserstoff oder eine Niedrigalkylgruppe mit 1 bis 4 C-Atomen bedeutet, mit 1 bis 10 Mol Ethylenoxid und/oder Propylenoxid in Haarbehandlungsmitteln als Zusatz zur Verbesserung der Naßkämmbarkeit der Haare.

Die Umsetzung der Organophosphonsäuren der Formel $R—PO_3H_2$ mit Ethylenoxid und/oder Propylenoxid erfolgt nach an sich literaturbekannten Verfahren, wie sie z.B. im « Surface Active Ethlene Oxide Adducts » von N. Schönfeld, Pergammon Press, 1969 angegeben sind.

Zur Anlagerung von Ethylenoxid oder Propylenoxid an die aziden OH-Gruppen der Phosphonatgruppen genügt es, die Phosphonsäure in einem Druckgefäß bei 120 bis 180 °C mit Ethylenoxid und/oder Propylenoxid umzusetzen. Zur Anlagerung weiteren Ethylenoxids oder Propylenoxids an die dabei gebildeten Hydroxyethyl- oder Hydroxypropylgruppen oder an weitere, im Molekül vorhandene, weniger azide Hydroxylgruppen ist es erforderlich, katalytische Mengen von Alkali, z.B. von Natriumalkoholat, Kaliumhydroxid oder Calciumacetat entweder der Organophosphonsäure vor der Alkoxylierung oder

nach Umsetzung der aziden OH-Gruppen zuzugeben. Eine weitere Möglichkeit der Umsetzung der Organophosphonsäuren mit Ethylenoxid und/oder Propylenoxid besteht darin, daß man die Organophosphonsäuren in Form der wässrigen Lösung ihres Alkalisalzes im Druckgefäß bei 120 bis 180 °C mit Ethylenoxid und/oder Propylenoxid zur Umsetzung bringt.

Bei der Umsetzung der Organophosphonsäuren mit Ethylenoxid entstehen Ethylenglycolester der Organophosphonsäuren. Bei Anlagerung weiteren Ethylenoxids an die Hydroxyethylgruppen der Ethylenglycolester bilden sich Polyethylenglycolester.

Entsprechend bilden sich bei der Umsetzung mit Propylenoxid Propylenglycolester bzw. Polypropylenglycolester.

Die zur Herstellung der erfindungsgemäßen Phosphonsäureester geeigneten Organophosphonsäuren R $PO_3H_2$ sind bekannte Verbindungen oder nach literaturbekannten Verfahren herstellbar. Zu ihnen gehören z. B. die Alkan-1-phosphonsäuren mit 2 bis 22 C-Atomen, die gemäß US-PS 2,957,931 durch radikalische Anlagerung von Estern der phosphorigen Säure an Olefine und nachfolgende Verseifung hergestellt werden können.

Alkan-1,1-diphosphonsäuren können z. B. durch Alkylierung von Methandiphosphonsäure nach dem Verfahren hergestellt werden, welches G.M. Kosoapoff in J. Am. Chem. Soc. 75 (1953), Seite 1500-1501 für die Pentan-1.1-diphosphonsäure angegeben hat.

1-Hydroxyalkan-1-phosphonsäuren können z. B. nach dem von W. Fossek in Monatshefte für Chemie, Bd. 7, (1886), Seite 20-39 angegebenen Verfahren durch Umsetzung von Aldehyden mit $PCl_3$ dargestellt werden.

1-Hydroxyalkan-1.1-diphosphonsäuren sind nach den von B. Blaser et al. in der Zeitschrift für anorganische und allgemeine Chemie, Bd. 381 (1971), Seite 247-259 beschriebenen Verfahren aus Carbonsäuren, Wasser und $PCl_3$ zugänglich.

1-Aminoalkan-1.1-diphosphonsäuren können nach einem von W. Plöger et al. in der Zeitschrift für anorganische und allgemeine Chemie, Bd. 389 (1972), Seite 119-128 beschriebenen Verfahren hergestellt werden.

Nach dem dort beschriebenen Verfahren sind auch die Verbindungen in welchen R der allgemeinen Formel III entspricht, die N-monosubstituierten und N,N-disubstituierten Aminomethan-diphosphonsäuren, zugänglich.

Die Verbindungen, in welchen R der allgemeinen Formel II entspricht, sind aus dem entsprechenden Alkylamin, Formaldehyd und phosphoriger Säure nach dem aus DE-AS 12 14 229 bekannten Verfahren herstellbar.

Bevorzugt geeignete Phosphonsäureester sind solche Verbindungen, die aus Organophosphonsäuren R $PO_3H_2$, in welchen R eine lineare Alkylgruppe mit 8 bis 18 C-Atomen ist, durch Umsetzung mit 1 bis 5 Mol Ethylenoxid und/oder Propylenoxid zugänglich sind. Besonders wirksam sind z. B. das Anlagerungsprodukt von 1 Mol Ethylenoxid an n-Octanphosphonsäure und das Anlagerungsprodukt von 1 Mol Ethylenoxid an n-Octadecanphosphonsäure.

Weitere, bevorzugt geeignete Phosphonsäureester sind solche Verbindungen, die aus Organophosphonsäuren R $PO_3H_2$, in welchen R eine Gruppe der allgemeinen Formel I ist, in der $R^1$ eine Methylgruppe $R^2$ eine $PO_3H_2$-Gruppe und $R^3$ eine OH-Gruppe darstellt, durch Umsetzung mit 2 bis 8 Mol Ethylenoxid und/oder Propylenoxid zugänglich sind. Besonders wirksam sind z. B. die Anlagerungsprodukte von 1-Hydroxyethan-1.1-diphosphonsäure mit 2 bis 6 Mol Propylenoxid.

Gut wirksam sind auch solche Phosphonsäureester, die aus Organophosphonsäuren R $PO_3H_2$, in welchen R eine Gruppe der allgemeinen Formel II ist, in der $R^4$ eine $PO_3H_2$-Gruppe ist, durch Umsetzung mit 3 bis 6 Mol Ethylenoxid und/oder Propylenoxid zugänglich sind.

Geeignet sind auch Phosphonsäureester, die durch gleichzeitige oder aufeinanderfolgende Umsetzung der Organophosphonsäuren mit Ethylenoxid und Propylenoxid erhalten werden.

Die erfindungsgemäß zu verwendenden Phosphonsäureester lösen sich in Wasser und in wässrigen Zubereitungen leicht auf. Wässrige Zubereitungen der Phosphonsäureester haben eine starke, die Kämmbarkeit des Haares, vor allem die Naßkämmbarkeit verbessernde Wirkung. Bereits geringe Zusätze der Phosphonsäureester zu Haarbehandlungsmitteln, insbesondere zu wässrigen, tensidhaltigen Zubereitungen, verbessern daher die Kämmbarkeit des Haares im nassen Zustand. Gleichzeitig wird eine verringerte statische Aufladbarkeit und eine bessere Frisierbarkeit — also ein konditionierender Effekt — für das getrocknete Haar erreicht. Die Phosphonsäureester führen dabei nicht zu einer Akkumulation auf der Haaroberfläche mit dem damit verbundenen, unerwünschten, beschwerenden Effekt. Die Phosphonsäureester können daher Haarpflege- und Haarbehandlungsmitteln wie z. B. Shampoos, Haarnachbehandlungsmitteln, Haarkurpräparaten, Haarfärbemitteln, Haarbleichmitteln, Dauerwellmitteln, Dauerwellfixierlösungen u.s.w. in einem weiten Konzentrationsbereich von 0,1 bis 10 Gewichtsprozent zugesetzt werden. Eine Menge von 0,1 bis 5 Gewichtsprozent ist aber in den meisten Fällen für eine befriedigende Wirkung ausreichend.

Die erfindungsgemäß zu verwendenden Phosphonsäureester sind auch mit den typischen, für die Formulierung von Haarbehandlungsmitteln geeigneten und bekannten Komponenten problemlos verträglich. Solche Komponenten sind vor allem anionische Tenside, z. B. Alkylsulfate und/oder Alkylpolyglycolethersulfate mit 10 bis 18 C-Atomen in der Alkylgruppe und bis zu 12 Polyglycolethergruppen und/oder Alkylpolyglycolether-sulfobernsteinsäuremonoester mit 10 bis 16 C-Atomen in der Alkylgruppe und 2 bis

6 Glycolethergruppen. Weitere geeignete Aniontenside für die Herstellung erfindungsgemäßer Haarbehandlungsmittel sind primäre und sekundäre lineare Alkansulfonate mit 10 bis 18 C-Atomen, Alkensulfonate und Hydroxyalkansulfonate, wie sie bei der Sulfonierung von Olefinen mit 10 bis 18 C-Atomen erhalten werden, Fettsäurealkylolamid- und Fettsäurealkylolamidpolyglycolethersulfate, sulfatierte Fettsäuremonoglyceride, Alkylpolyglykolethercarboxylate mit 8 bis 18 C-Atomen in der Alkylkette und 1 bis 10 Glycolethergruppen, Acylsarkosine, Acyltauride und Acylisethionate mit 8 bis 18 C-Atomen in der Acylgruppe.

Die genannten anionischen Tenside können in Form der Alkali-, Ammonium-, Alkanolammoniumsalze, die Alkylsulfate und Alkylpolyglycolethersulfate auch in Form der Magnesiumsalze vorliegen. Ihre Menge beträgt üblicherweise 2 bis 50 Gewichtsprozent des Haarbehandlungsmittels.

Eine besonders ausgeprägte Senkung des Kämmwiderstandes wurde bei Haarbehandlungsmitteln gefunden, welche die erfindungsgemäßen Phosphonsäureester und als anionische Tenside ein Alkylpolyglycolethersulfat-Alkalisalz mit 10 bis 16 C-Atomen in der Alkylgruppe und 2 bis 4 Glycolethergruppen enthalten.

Besonders geeignet sind die Phosphonsäureester zur Formulierung von konditionierenden Shampoos auf Basis von anionischen Tensiden. Solche Shampoos enthalten neben 5 bis 20 Gewichtsprozent anionischer Tenside bevorzugt 1 bis 10 Gewichtsprozent ampholytische Tenside, Betaintenside oder gegebenenfalls auch Aminoxid-Tenside. Als anionische Tenside kommen die vorgenannten Produkte, insbesondere aber Alkylpolyglycolethersulfate in Frage.

Als ampholytische Tenside können z. B. N-$C_8$-$C_{18}$-Alkyl-ß-aminopropionsäuren, N-$C_8$-$C_{18}$-Alkyl-ß-iminodipropionsäuren oder N-Hydroxyethyl-N-Kokosamidopropyl-glycin eingesetzt werden. Geeignete Betaintenside sind z. B. N-Kokosalkyl-N.N-dimethyl-glycin und N-Kokosamidopropyl-N.N-dimethyl-glycin. Als Amin-oxid-Tenside sind z. B. N-Kokosamidopropyl-N.N-dimethylamin-oxid oder N-Kokosalkyl-N.N-di(2-hydroxy)ethylamin-oxid geeignet.

Solche Shampoos auf Basis von anionischen und bevorzugt ampholytischen Tensiden oder Betaintensiden enthalten zur Verbesserung der Naßkämmbarkeit und der konditionierenden Wirkung einen Zusatz von wenigstens einem erfindungsgemäßen Phosphonsäureester bevorzugt in einer Menge von 1 bis 5 Gewichtsprozent.

Neben den genannten Tensiden können die erfindungsgemäßen Haarshampoos auch weitere übliche Hilfs- und Zusatzstoffe wie z. B. nichtionogene Tenside, geringe Mengen kationischer Tenside, Fettsäurealkanolamide, wasserlösliche Polymere wie z. B. Celluloseether und Carboxyvinylpolymere, Puffersubstanzen, Konservierungsstoffe, Farbstoffe, Duftstoffe, haarkosmetische Wirkstoffe wie. z. B. Antischuppenwirkstoffe, Sebostatika, Vitamine, Kräuterextrakte u.s.w. enthalten.

Die nachfolgenden Beispiele sollen den Gegenstand der Erfindung näher erläutern ohne ihn hierauf zu beschränken.

## Beispiele

### 1. Herstellung der Phosphonsäureester

#### 1.1 n-Octanphosphonsäure-ethylenglycolester

485 g n-Octanphosphonsäure (2,5 Mol) wurden in einem Druckgefäß portionsweise mit 110 g Ethylenoxid (2,5 Mol) bei 138 °C und einem Druck von 6 bar 2 Stunden zur Umsetzung gebracht. Es wurde ein wasserhelles Öl mit folgenden Kennzahlen erhalten :

| | |
|---|---|
| Dichte $D_{20}$ | 1.082 |
| Brechungsindex $n_D$ | 1.4547 |

#### 1.2 n-Octanphosphonsäurepoly(3 EO)ethylenglycolester

291 g n-Octanphosphonsäure (1,5 Mol) wurden mit 4 g einer 30 gewichtsprozentigen Lösung von Natriummethylat in Methanol in einem Druckgefäß vorgelegt. Dann wurde ein Vakuum von ca. 10 mbar angelegt und ca. 30 Minuten bei 100 °C evakuiert. Dann wurde die Temperatur auf 170 °C erhöht und bei einem Druck von 10 bar in ca. 2 Stunden 198 g gasförmiges Ethylenoxid (4,5 Mol) eingeleitet. Es wurde ein wasserhelles Öl mit folgenden Kennzahlen erhalten :

| | |
|---|---|
| Dichte | 1.096 |
| Brechungsindex | 1.4593 |

#### 1.3 1-Hydroxyethan-1.1-diphosphonsäure-poly (4 PO)-propylenglycolester

250 g 1-Hydroxyethan-1.1-diphosphonsäure-di-Na-Salz (1 Mol) wurde in 192 g Wasser gelöst in einem Druckgefäß vorgelegt. In die auf 145 °C erwärmte Lösung wurden in ca. 5 Stunden 232 g Propylenoxid (4 Mol) bei einem Druck von max. 8 bar eingeleitet. Es wurde eine bei Raumtemperatur erstarrende,

kristalline Masse erhalten, die ca. 71,5 Gewichtsprozent Trockensubstanz enthielt und in Wasser leicht löslich war.

2. Prüfung der Naßkämmbarkeit der Haare NK [%]

Die Wirkung der Zusätze von Phosphonsäurepolyglycolestern auf die Naßkämmbarkeit der Haare wurde nach folgender Methode geprüft :

0,8 Gramm schwere, ca. 11 cm lange braune, europäische, durch einmalige Blondierung und Kaltwelle definiert vorbehandelte Haarsträhnen wurden mit einer Lösung von 2,0 Gew.-% der Phosphonsäurepolyglycolester in einer wässrigen Tensidlösung 5 Minuten lang bei 30 °C behandelt und anschließend in lauwarmem Wasser (30 °C) gründlich ausgespült und überschüssiges Wasser abgestreift. Sodann erfolgte die Messung des Kämmwiderstandes, d. h. der Kraft, die erforderlich war, um einen Kamm durch eine Haarsträhne zu ziehen. Zur Verringerung des Meßfehlers wurde die Bestimmung 15-fach mit jedem der zu prüfenden Produkte durchgeführt und der Mittelwert der Arbeitsintegrale gebildet. Die Messung erfolgte in einer Zug-Prüfmaschine des Typs 1402 der Fa. Zwick (Einsingen über Ulm/Donau).

Das mittlere Arbeitsintegral wurde auf das ohne Wirkstoff durch Behandlung mit der jeweiligen Tensidlösung erhaltene Arbeitsintegral (Blindwert) bezogen und zeigte so die Naßkämmbarkeitsverbesserung (oder Verschlechterung) :

$$NK\,[\%] = \frac{\text{Arbeitsintegral Wirkstoff}}{\text{Arbeitsintegral Blindwert}} \cdot 100$$

Werte unter 100 % bedeuten demnach eine Verbesserung, Werte über 100 % eine Verschlechterung der Naßkämmbarkeit der Haare.

Die Ergebnisse für erfindungsgemäß in Haarbehandlungsmitteln einzusetzenden Phosphonsäureester zeigt Tabelle I.

(Siehe Tabelle 1 Seite 6 ff.)

Tabelle 1

| Nr. | Phosphonsäure-ester (2 % AS) | Tensid (14 % AS) | NK (%) |
|---|---|---|---|
| 2.1 | n-Octanphosphonsäure + 1 EO x) | Fettalkohol C 12/14 + 2 EO-sulfat, Na-Salz | 63 |
| 2.2 | n-Octanphosphonsäure + 1 EO | Laurylsulfat, triethanolammonium-Salz | 77 |
| 2.3 | n-Octanphosphonsäure + 1 EO | N-Kokosamidopropyl-N.N-dimethyl-glycin | 84 |
| 2.4 | n-Octanphosphonsäure + 1 EO | Fettalkohol C 12/14 + 3 EO-sulfo-bernsteinsäuremonoester, Na-Salz | 82 |
| 2.5 | n-Octanphosphonsäure + 3 EO | Fettalkohol C 12/14 + 2 EO-sulfat, Na-Salz | 87 |
| 2.6 | n-Decanphosphonsäure + 1 EO | Fettalkohol C 12/14 + 2 EO-sulfat, Na-Salz | 73 |
| 2.7 | n-Octadecanphosphonsäure + 1 EO | Fettalkohol C 12/14 + 2 EO-sulfat, Na-Salz | 53 |
| 2.8 | 1-Hydroxyethan-1.1-di-phosphonsäure + 2 PO x) | Fettalkohol C 12/14 + 2 EO-sulfat, Na-Salz | 71 |
| 2.9 | 1-Hydroxyethan-1.1-di-phosphonsäure + 4 PO | Fettalkohol C 12/14 + 3 EO-sulfat, Na-salz | 69 |

Tabelle 1 (Fortsetzung)

| Nr. | Phosphonsäure-ester (2 % AS) | Tensid (14 % AS) | NK (%) |
|---|---|---|---|
| 2.10 | 1-Hydroxyethan-1.1-di-phosphonsäure + 4[x] PO | n-Kokosalkylamidopropyl-N.N-dimethyl-glycin | 78 |
| 2.11 | 1-Hydroxyethan-1.1-di-phosphonsäure + 6 PO | Fettalkohol C 12/14 + 2 EO-sulfat, Na-Salz | 70 |
| 2.12 | 1-Hydroxyethan-1.1-di-phosphonsäure + 4 EO | Fettalkohol C 12/14 + 2 EO-sulfat, Na-Salz | 85 |
| 2.13 | Aminotrimethylenphosphonsäure + 3 PO | Fettalkohol C 12/14 + 2 EO-sulfat, Na-Salz | 82 |

x EO bedeutet Mol angelagertes Ethylenoxid
PO bedeutet Mol angelagertes Propylenoxid

3. Anwendungsbeispiele

Haarshampooformulierungen

| Beispiel Nr. | 3.1 | 3.2 | 3.3 | 3.4 | 3.5 | 3.6 | 3.7 | 3.8 |
|---|---|---|---|---|---|---|---|---|
| Komponenten (Gew. %) | | | | | | | | |
| Fettalkohol ($C_{12}/_{14}$)-poly (2 EO)-glycolethersulfat, Na-Salz (28 %ig) (Texapon® N 25) | 50 | 40 | 45 | 40 | - | 50 | 40 | - |
| Fettalkohol ($C_{12}/_{14}$)-sulfat-triethanolaminsalz (42 %ig) (Texapon® T 42) | - | - | - | - | 30 | - | - | - |
| Gemisch aus Fettalkohol-Ethersulfaten und Sulfobernsteinsäurehalbestern (Na-Salze) 29 %ig (Texapon® SBN) | - | - | - | - | - | - | - | 40 |

| Beispiel Nr. | 3.1 | 3.2 | 3.3 | 3.4 | 3.5 | 3.6 | 3.7 | 3.8 |
|---|---|---|---|---|---|---|---|---|
| Komponenten (Gew. %) | | | | | | | | |
| N-Kokosamidopropyl-N.N-dimethyl-glycin (30 %ig) (Dehyton ® K) | - | - | 10 | - | - | - | 5 | - |
| N-Kokosamidoethyl-N-hydroxyethyl-glycin (30 %ig) (Dehyton ® G) | - | - | 5 | - | - | - | - | - |
| Phosphonsäurepolyglycolester nach | | | | | | | | |
| Beispiel 1.1 | 2 | 5 | 2,5 | 2 | 3 | - | - | - |
| Beispiel 1.2 | - | - | - | - | - | - | - | 2 |
| Beispiel 1.3 | - | - | - | - | - | 2.8 | 5.6 | - |
| Konservierungsmittel (Bronidox ® L) | 0.2 | 0.2 | 0.2 | 0.2 | 0.2 | 0.2 | 0.2 | 0.2 |
| | ad | ad | ad | ad | ad | ad | ad | ad |
| Wasser | 100 | 100 | 100 | 100 | 100 | 100 | 100 | 100 |

Die in der Tabelle aufgeführten Haarshampooformulierungen wurden durch Mischen der Komponenten bei Raumtemperatur (20 °C) hergestellt. Es handelt sich um klare und bei mehrwöchiger Lagerung bei + 5 °C klar bleibende Lösungen.

## Patentansprüche

1. Verwendung von Phosphonsäureestern, die erhältlich sind durch Umsetzung von Organophosphonsäuren der allgemeinen Formel

$$R—PO_3H_2$$

in der R eine lineare oder verzweigte Alkylgruppe mit 2 bis 22 C-Atomen oder eine Gruppe der allgemeinem Formeln I, II oder III

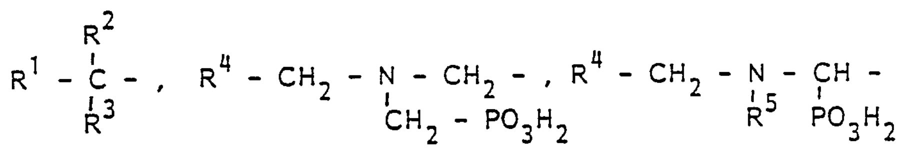

I II III

ist, in welchen $R^1$ Wasserstoff oder eine Alkylgruppe mit 1 bis 21 C-Atomen, $R^2$ Wasserstoff oder eine $PO_3H_2$-Gruppe, $R^3$ Wasserstoff, eine OH-Gruppe oder, wenn $R^2$ eine $PO_3H_2$-Gruppe ist, eine $NH_2$-Gruppe, $R^4$ eine Gruppe $R^1$ oder eine $PO_3H_2$-Gruppe und $R^5$ Wasserstoff oder eine Niedrigalkylgruppe mit 1 bis 4 C-Atomen bedeutet, mit 1 bis 10 Mol Ethylenoxid und/oder Propylenoxid in Haarbehandlungsmitteln als Zusatz zur Verbesserung der Naßkämbarkeit der Haare.

2. Verwendung nach Anspruch 1, dadurch gekennzeichnet, daß R eine lineare Alkylgruppe mit 6 bis 18 C-Atomen ist und die Umsetzung mit 1 bis 5 Mol Ethylenoxid und/oder Propylenoxid erfolgt ist.

3. Verwendung nach Anspruch 1, dadurch gekennzeichnet, daß R eine Gruppe der allgemeinen Formel I ist, in der $R^1$ eine Methylgruppe, $R^2$ eine $PO_3H_2$-Gruppe und $R^3$ eine OH-Gruppe darstellt und die Umsetzung mit 2 bis 8 Mol Ethylenoxid und/oder Propylenoxid erfolgt ist.

4. Verwendung nach Anspruch 1, dadurch gekennzeichnet, daß R eine Gruppe der allgemeinen Formel II ist, in der $R^4$ eine $PO_3H_2$-Gruppe darstellt und die Umsetzung mit 3 bis 6 Mol Ethylenoxid oder Propylenoxid erfolgt ist.

5. Haarshampoos mit einem Gehalt anionischer und bevorzugt ampholytischer Tenside oder Betaintenside, dadurch gekennzeichnet, daß zu Verbesserung der Naßkämmabarkeit des Haares ein Zusatz von wenigstens einem Phosphonsäureester, der erhältlich ist durch Umsetzung von Organophosphonsäuren der allgemeinen Formel

$$R—PO_3H_2$$

in der R eine lineare oder verzweigte Alkylgruppe mit 2 bis 22 C-Atomen oder eine Gruppe der allgemeinen Formeln I, II oder III

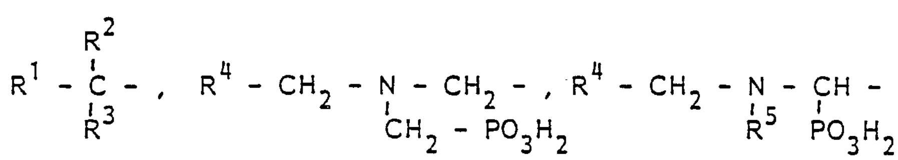

I II III

ist, in welchen $R^1$ Wasserstoff oder eine Alkylgruppe mit 1 bis 21 C-Atomen, $R^2$ Wasserstoff oder eine $PO_3H_2$-Gruppe, $R^3$ Wasserstoff, eine OH-Gruppe oder, wenn $R^2$ eine $PO_3H_2$-Gruppe ist, eine $NH_2$-Gruppe, $R^4$ eine Gruppe $R^1$ oder eine $PO_3H_2$-Gruppe und $R^5$ Wasserstoff oder eine Niedrigalkylgruppe mit 1 bis 4 C-Atomen bedeutet, mit 1 bis 10 Mol Ethylenoxid und/oder Propylenoxid in einer Menge von 1-5 Gew.% enthalten ist.

## Claims

1. The use of phosphonic acid esters obtainable by reaction of organophosphonic acids corresponding to the general formula

$R—PO_3H_2$

in which R is a linear or branched alkyl group containing 2 to 22 C atoms or a group corresponding to general formulae I, II or III

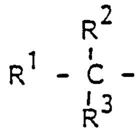 , 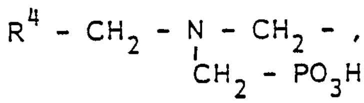 , 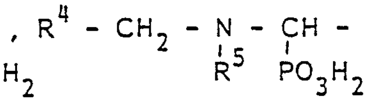

I II III

in which $R^1$ is hydrogen or an alkyl group containing 1 to 21 C atoms, $R^2$ is hydrogen or a $PO_3H_2$ group, $R^3$ is hydrogen, an OH group or, where $R^2$ is a $PO_3H_2$ group, represents an $NH_2$ group, $R^4$ is a group $R^1$ or a $PO_3H_2$ group and $R^5$ is hydrogen or a lower alkyl group containing 1 to 4 C atoms, with from 1 to 10 mol ethylene oxide and/or propylene oxide in hair treatment preparations as an additive for improving the wet combability of hair.

2. The use claimed in claim 1, characterized in that R is a linear alkyl group containing 6 to 18 C atoms and the reaction is carried out with 1 to 5 mol ethylene oxide and/or propylene oxide.

3. The use claimed in claim 1, characterized in that R is a group corresponding to general formula I, in which $R^1$ is a methyl group, $R^2$ is a $PO_3H_2$ group and $R^3$ is an OH group and the reaction is carried out with 2 to 8 mol ethylene oxide and/or propylene oxide.

4. The use claimed in claim 1, characterized in that R is a group corresponding to general formula II, in which $R^4$ is a $PO_3H_2$ group and the reaction is carried out with 3 to 6 mol ethylene oxide or propylene oxide.

5. Hair shampoos containing anionic and, preferably, ampholytic surfactants or betaine surfactants, characterized in that, to improve the wet combabaility of the hair, they contain an addition of at least one phosphonic acid ester obtainable by reaction of organophosphonic acids corresponding to the following general formula

$R—PO_3H_2$

in which R is a linear or branched alkyl group containing from 2 to 22 C atoms or a group corresponding to general formulae I, II or III below

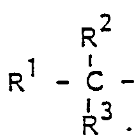 , 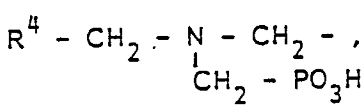 , 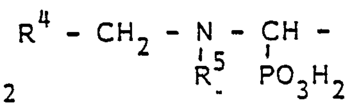

I II III

in which $R^1$ is hydrogen or an alkyl group containing from 1 to 21 C atoms, $R^2$ is hydrogen or a $PO_3H_2$ group, $R^3$ is hydrogen, an OH group or, where $R^2$ is a $PO_3H_2$ group, represents an $NH_2$ group, $R^4$ is a group $R^1$ or a $PO_3H_2$ group and $R^5$ is hydrogen or a lower alkyl group containing 1 to 4 C atoms, with 1 to 10 mol ethylene oxide and/or propylene oxide in a quantity of from 1 to 5 % by weight.

**Revendications**

1. Utilisation dans des produits de traitement des cheveux, en qualité d'additifs destinés à améliorer la « coiffabilité » des cheveux mouillés, d'esters d'acide phosphonique, obtenables en faisant réagir avec 1 à 10 moles d'oxyde d'éthylène et/ou d'oxyde de propylène des acides organophosphoniques de formule générale :

$R—PO_3H_2$

dans laquelle R représente un groupement alkyle linéaire ou ramifié comportant 2 à 22 atomes de carbone, ou un groupement des formules générales I, II ou III

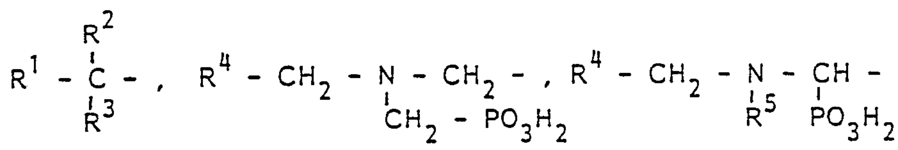

I II III

dans lesquelles $R^1$ est de l'hydrogène ou un groupement alkyle comportant 1 à 21 atomes de carbone, $R^2$ de l'hydrogène ou un groupement $PO_3H_2$, $R^3$ de l'hydrogène, un groupement OH ou, si $R^2$ est un groupement $PO_3H_2$, un groupement $NH_2$, $R^4$ un groupement $R^1$ ou un groupement $PO_3H_2$, et $R^5$ de l'hydrogène ou un groupement alkyle inférieur comportant 1 à 4 atomes de carbone.

2. Utilisation selon la revendication 1, caractérisée en ce que R est un groupement alkyle linéaire comportant 6 à 18 atomes de carbone et en ce que la réaction s'effectue avec 1 à 5 moles d'oxyde d'éthylène ou d'oxyde de propylène.

3. Utilisation selon la revendication 1, caractérisée en ce que R est un groupement de la formule générale I, dans laquelle $R^1$ représente un groupement méthyle, $R^2$ un groupement $PO_3H_2$ et $R^3$ un groupement OH et en ce que la réaction s'effectue avec 2 à 8 moles d'oxyde d'éthylène et/ou d'oxyde de propylène.

4. Utilisation selon la revendication 1, caractérisée en ce que R est un groupement de la formule générale II, dans laquelle $R^4$ représente un groupement $PO_3H_2$ et en ce que la réaction s'effectue avec 3 à 6 moles d'oxyde d'éthylène et/ou d'oxyde de propylène.

5. Shampooings renfermant des tensioactifs anioniques et de préférence ampholytes ou bétaïnes, caractérisés en ce qu'ils renferment pour améliorer la « coiffabilité » des cheveux mouillés un additif en proportion de 1 à 5 % en poids d'au moins un ester d'acide phosphonique obtenable par mise en réaction avec 1 à 10 moles d'oxyde d'éthylène et/ou d'oxyde de propylène d'acides organophosphoniques de formule générale

$$R—PO_3H_2$$

dans laquelle R est un groupement alkyle linéaire ou ramifié comportant 2 à 22 atomes de carbone ou un groupement des formules générales I, II ou III

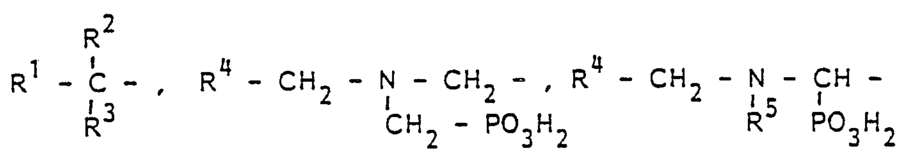

I II III

dans lesquelles $R^1$ est de l'hydrogène ou un groupement alkyle comportant 1 à 21 atomes de carbone, $R^2$ de l'hydrogène ou un groupement $PO_3H_2$, $R^3$ de l'hydrogène, un groupement OH ou si $R^2$ est un groupement $PO_3H_2$, un groupement $NH_2$, $R^4$ un groupement $R^1$ ou un groupement $PO_3H_2$, et $R^5$ de l'hydrogène ou un groupement alkyle inférieur comportant 1 à 4 atomes de carbone.